Europäisches Patentamt

European Patent Office

Office européen des brevets

(11) Publication number: **0 358 302**
**A2**

## EUROPEAN PATENT APPLICATION

(21) Application number: 89305170.6

(22) Date of filing: 23.05.89

(51) Int. Cl.⁵: **A61M 1/00**

The title of the invention has been amended (Guidelines for Examination in the EPO, A-III, 7.3).

(30) Priority: 28.05.88 GB 8812803

(43) Date of publication of application:
**14.03.90 Bulletin 90/11**

(84) Designated Contracting States:
**AT BE CH DE ES FR GR IT LI LU NL SE**

(71) Applicant: **Smiths Industries Public Limited Company**
**765, Finchley Road**
**London, NW11 8DS(GB)**

(72) Inventor: **Wood, James Michael**
**5 The Grovelands**
**Lancing West Sussex BN15 8HY(GB)**

(74) Representative: **Flint, Jonathan McNeill**
**SMITHS INDUSTRIES PUBLIC LIMITED**
**COMPANY 765 Finchley Road**
**London NW11 8DS(GB)**

(54) Medico-surgical suction container.

(57) A medico-surgical suction container has an inlet 10 connected to a suction catheter 11 and an outlet 20 connected to a pump 21 capable of reducing pressure in the container to at least 500 mm Hg below atmosphere. Within the container, in line with the outlet 20, is a housing 44 containing a filter 41 having a layer of a PTFE membrane on a support screen and a layer of a glass microfibre laminated to a polymer monofilament. The filter 41 allows passage of gas from the container but prevents passage of bacteria and liquid. A tube 43 projects down from the filter housing 44 into the container, the lower end of which defines the maximum filling level, thereby preventing overfilling of the container. The inlet 10 and outlet 20 are formed in recesses 13 and 23 which can be sealed by plugs 14 and 24 attached to the container by flexible webs 15 and 25. The plugs 14 and 24, when inserted, form a smooth surface of the recesses, thereby preventing subsequent removal. An expansion chamber 30 has a convex base plate 31 beneath the inlet 10 so that liquid flows outwardly and down the edge of the plate between a gap 32 with the container and through slots 33 at the edge of the plate.

Fig.1.

# MEDICO-SURGICAL CONTAINERS

This invention relates to medico-surgical containers of the kind having an inlet for connection to a suction catheter and an outlet for connection to a vacuum pump.

The invention is more particularly concerned with containers for collecting liquid and other debris removed from a surgical site by suction in a suction system.

Suction applied by a vacuum pump is used to remove blood, irrigation liquid, tissue debris and the like during surgery. The suction system commonly comprises a suction catheter, a vacuum pump and a collection container. The collection container has two openings one of which is connected to the suction catheter and the other of which is connected to the vacuum pump. The reduced pressure produced by the vacuum pump is communicated with the suction catheter via the container so that material in the surgical site can be sucked along the catheter and collected in the container.

Such collection containers usually have a float-actuated ball valve in the outlet connected to the vacuum pump, the ball valve closing when the liquid in the container rises above a preset level, so as to prevent the liquid being sucked into the vacuum pump. A bacterial filter is often connected between the container and the pump to prevent any airborne or aerosol-borne bacteria being dispersed to the atmosphere.

Because the contents of the collection container after use are often contaminated, their safe disposal presents problems.

It is an object of the present invention to provide an improved medico-surgical container.

According to the present invention there is provided a medico-surgical container of the above-specified kind, characterised in that the container includes a filter located in the container in line with the outlet and that the filter allows passage of gas from the container to the outlet but prevents passage of bacteria and liquid such that overfilling of the container is prevented by the filter.

In this way, the need for a separate float valve and bacterial filter is obviated.

The filter is preferably contained within a housing having a tube projecting downwardly into the container, the lower end of the tube defining the maximum filling level of the container. The filter may include a layer comprising a PTFE membrane on a support screen and may include a layer including a glass microfibre laminated to a polymer monofilament.

The inlet and outlet may be located in recesses, the container including plugs shaped for insertion in the recesses to seal off the inlet and outlet and to form a smooth surface of the recess making subsequent removal of the plug difficult. The plugs may be each attached to the container by means of a flexible web.

The container preferably includes an expansion chamber beneath the inlet. The expansion chamber may have a base plate of convex shape located such that the liquid from the inlet flows outwardly and down the edge of the plate. The outlet from the expansion chamber is preferably at the edge of the base plate and may include slots formed at the edge of the base plate.

A suction system including a suction container in accordance with the present invention will now be described, by way of example, with referene to the accompanying drawings.

Figure 1 is a sectional side elevation view of the container;

Figure 2 is a perspective view of the top of the container;

Figure 3 is a perspective view of the interior of the top of the container; and

Figure 4 is a sectional elevation through the filter member of the container.

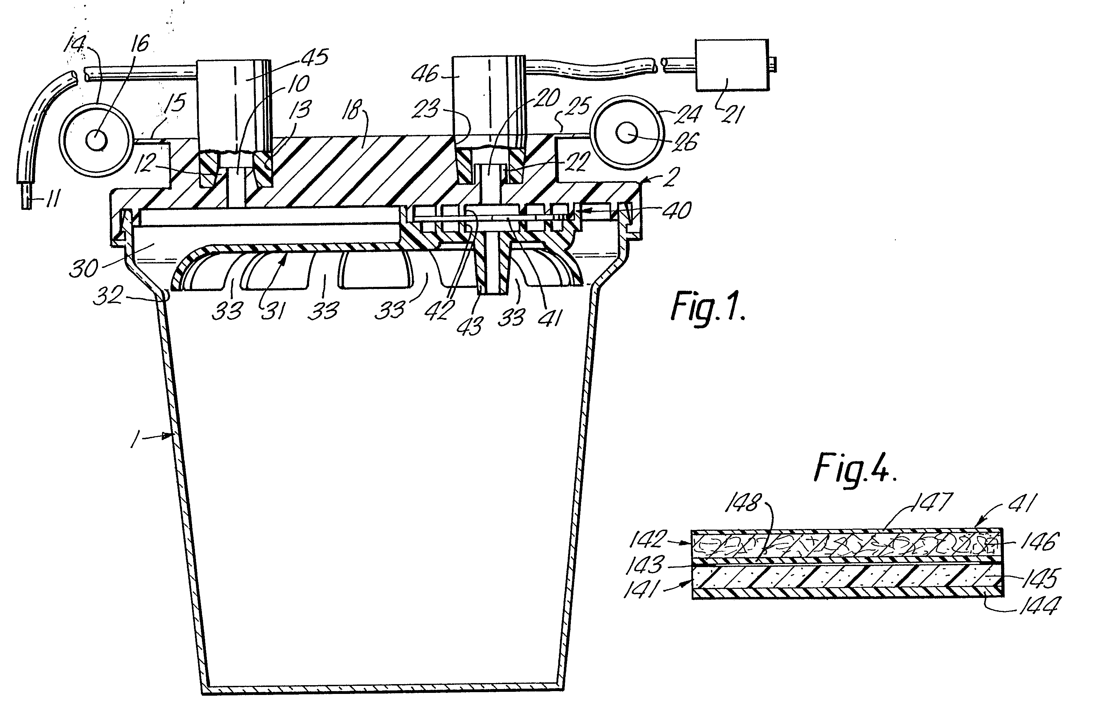

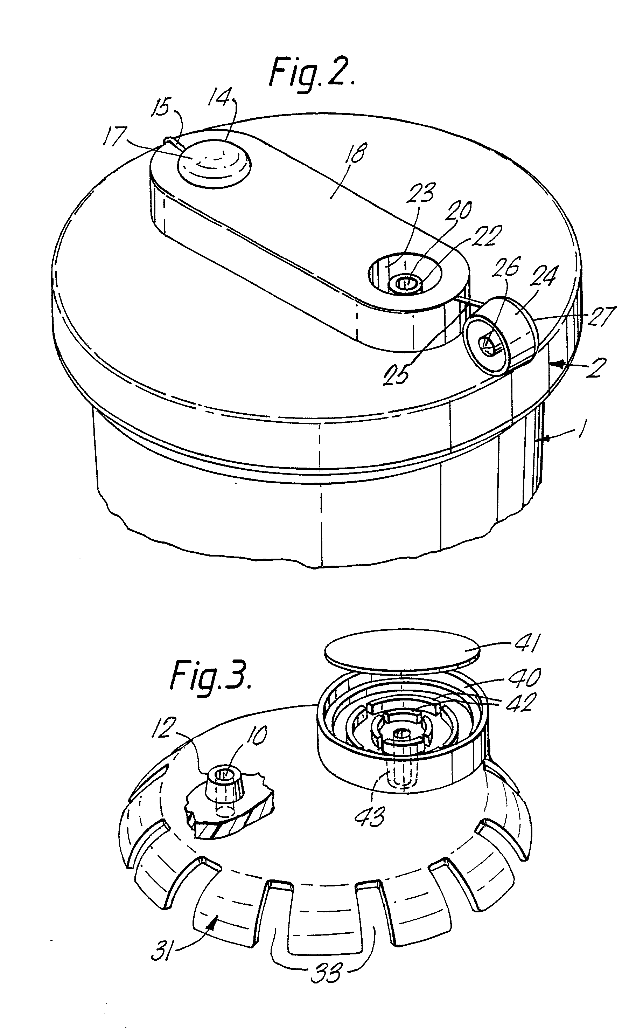

With reference first to Figures 1 and 2, the surgical suction system includes a container comprising a cylindrical, transparent jar 1 of a plastics or glass and a top closure 2 that is irremovably sealed to the jar. An inlet 10 and outlet 20 are provided in the top closure 2 that are connected respectively to a suction catheter 11 and a vacuum pump 21 capable of delivering a vacuum of at least 500 mm Hg below atmosphere in the jar 1.

The inlet 10 and outlet 20 both have a vertical spigot 12 and 22 located within respective cylindrical recesses 13 and 23 formed in a handle 18 that extends across the top of the closure 2. The inlet spigot 12 has a tapered outer surface which mates with a female connector 45 that is connected to the suction catheter 11. The outlet recess 23 is tapered outwardly towards its upper end to receive therein, as a mating fit, a male connector 46 that is connected to the vacuum pump 21. The connectors 45 and 46 are differently shaped and the recesses 13 and 23 are of different diameters so that it is not possible to fit the connectors into the wrong recesses. At opposite ends of the handle 18, plugs 14 and 24 are attached by means of short flexible webs 15 and 25, one of the plugs 14 being shown inserted in Figure 2. One side of each plug 14 and 24 is hollow and formed with a central nose 16 and 26, the external diameter of each plug being such that it is a close, push fit within the recess 13 and 23 with the nose being a close sealing fit within the respective spigot 12 and 22. The other side of each plug 14 and 24 has a shallow convex surface 17 and 27 respectively. The plugs 14 and 24 can be inserted into their adjacent recess 10 or 20 by bending and twisting their web 15 and 25 so that the convex surface 17 or 27 forms a smooth, shallow projection from the handle which cannot be gripped easily. This prevents the plugs being pulled out readily once inserted.

The inlet 10 opens into an expansion chamber 30 formed in the top closure 2 and seen most clearly in Figure 3. The chamber 30 has a base plate 31 of convex shape which underlies the inlet 10 so that liquid from the inlet flows outwardly and down the edge of the plate. The diameter of the plate 31 is slightly less than that of the jar 1 so that an annular gap is formed around the outer edge of the plate, between the inner surface of the jar, which provides an outlet from the chamber 30 offset laterally from its inlet 10. Radial slots 33 are spaced around the edge of the plate 31 to provide additional liquid flow paths into the jar. The purposes of the expansion chamber 30 is to reduce turbulence and splashing inside the jar 1 by providing an indirect and smooth flow of liquid from the expansion chamber into the jar.

The spigot 22 of the vacuum outlet 20 opens into a filter assembly 40 in the top closure 2. The filter assembly 40 has a housing 44 containing a hydrophobic filter element 41 of the kind sold by Arbor Technologies under the trade mark CONTAIN and the code number 85005. The element 41 is shown in more detail in Figure 4 and is a membrane made up of two layers 141 and 142 bonded together around their edge 143. The layer 141 facing the inside of the jar 1 comprises a PTFE membrane 144 laminated with a polypropylene support screen 145, the PTFE membrane facing outwardly. This layer is tested to withstand water breakthrough at at least 10 psi. The layer 142 facing the pump 21 is a glass microfibre 146 which is laminated on both sides with polypropylene monofilament 147 and 148 that is treated to render it hydrophobic. The element 41 is retention rated at a particle size of 0.3 micron and can withstand pressure across it of 700 mm Hg. Although different forms of filter element may be effective at removing bacteria at low pressure, at the relatively high pressures encountered in surgical suction systems, a membrane type of element, such as of the kind described, is most effective. The element 41 is supported on both sides by ribs 42 formed internally of the housing 44. On its lower side, the filter assembly 40 communicates with a short vertical vent tube 43 that projects downwardly of the housing 44 into the jar 1 by a short distance, the lower end of the vent tube defining the maximum filling volume of the jar. The element 41 thereby communicates with the jar 1 without the interposition of any separate value or overflow prevention device.

When the pump 21 is turned on, it draws air out of the container through the filter assembly 40 which acts as a bacterial filter to prevent contamination of the pump or atmosphere. The reduced pressure inside the container causes suction to be applied to the suction inlet 10 and hence to the suction catheter 11. This in turn causes any liquid or small debris in the region of the operative tip of the catheter 11 to be sucked along the catheter, through the inlet 10 and the expansion chamber 30 into the container until the level of contents in the jar 1 reaches the lower end of the vent tube 43. When this happens, liquid is drawn up the tube 43 into the filter assembly 40. Although the filter element 41 allows passage of gas, it prevents the passage of liquid, so that the contents of the container are prevented from reaching the vacuum outlet 20. Because further gas flow to the vacuum pump 21 is prevented, suction ceases at the catheter 11, signalling to the user that the container is full. The user then turns off the pump and disconnects the inlet 10 and outlet 20 from their connections, thereby allowing liquid in the filter assembly 40 and tube 43 to flow back down into the jar 1. The plugs 14 and 24 are then pushed into the respective recesses 13 and 23 to seal the jar 1 closed.

The hydrophobic filter serves the dual function of preventing overfilling and of removing bacteria from gas vented from the container. It avoids the need to provide a separate bacterial filter, thereby simplifying the setting up of the suction system. There is a risk, where a separate bacterial filter is used, that replacement of the filter will be overlooked and that a filter may be left in the system long enough to become damaged. In the present arrangement, because the filter is disposed of every time the collection jar is full, there is less risk of contamination caused by use of a damaged filter. The use of a membrane type filter element enables effective bacterial filtering at the relatively high pressure differentials of about 500 mm Hg encountered in surgical suction systems.

## Claims

1. A medico-surgical container having an inlet for connection to a suction catheter and an outlet for connection to a vacuum pump, characterised in that the container includes a filter (41) located in the container in line with the outlet (20), and that the filter (41) allows passage of gas from the container to the outlet (20) but prevents passage of bacteria and of liquid such that overfilling of the container is prevented by the filter (41).

2. A container according to Claim 1, characterised in that the filter (41) is contained within a housing (44) having a tube (43) projecting downwardly into the container, the lower end of the tube (43) defining the maximum filling level of the container.

3. A container according to Claim 1 or 2, characterised in that the filter (41) includes a layer comprising a PTFE membrane (144) on a support screen (145).

4. A container according to any one of the preceding claims, characterised in that the filter (41) includes a layer (142) including a glass microfibre (146) laminated to a polymer monofilament (147, 148).

5. A container according to any one of the preceding claims, characterised in that the inlet (10) and outlet (20) are located in recesses (13 and 23), and that the container includes plugs (14 and 24) shaped for insertion in the recesses (13 and 23) to seal off the inlet (10) and outlet (20) and to form a smooth surface of the recess making subsequent removal of the plug difficult.

6. A container according to Claim 5, characterised in that the plugs (14 and 24) are each attached to the container by means of a flexible web (15 and 25).

7. A container according to any one of the preceding claims, characterised in that the container includes an expansion chamber (30) beneath the inlet (10).

8. A container according to Claim 7, characterised in that the expansion chamber (30) has a base plate (31) of convex shape located such that liquid from the inlet (10) flows outwardly and down the edge of the plate (31).

9. A container according to Claim 8, characterised in that the outlet (32, 33) from the expansion chamber (30) is at the edge of the base plate (31).

10. A container according to Claim 9, characterised in that the outlet from the expansion chamber (30) includes slots (33) formed at the edge of the base plate (31).

**Fig.1.**

**Fig.4.**

EP 0 358 302 A2

Fig.2.

Fig.3.